(19) Europäisches Patentamt
European Patent Office
Office européen des brevets

(11) EP 1 611 883 A1

(12) **EUROPEAN PATENT APPLICATION**
published in accordance with Art. 158(3) EPC

(43) Date of publication:
**04.01.2006 Bulletin 2006/01**

(21) Application number: **04712156.1**

(22) Date of filing: **18.02.2004**

(51) Int Cl.:
***A61K 31/16*** *(1985.01)* ***A61K 31/155*** *(1985.01)*
***A61K 31/785*** *(1985.01)* ***A61K 9/08*** *(1974.07)*
***A61P 27/04*** *(2000.01)* ***A61P 31/04*** *(2000.01)*
***A61L 2/18*** *(1980.01)*

(86) International application number:
**PCT/JP2004/001768**

(87) International publication number:
**WO 2004/084877 (07.10.2004 Gazette 2004/41)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **26.03.2003 JP 2003086018**

(71) Applicant: **Menicon Co., Ltd.**
**Nagoya-shi, Aichi 460-0006 (JP)**

(72) Inventors:
- **TSUZUKI, Akira,**
**c/o Menicon Co, , Ltd.**
**Kasugai-shi,**
**Aichi 4870032 (JP)**
- **TANIKAWA, Sadayasu,**
**c/o Menicon Co, , Ltd.**
**Kasugai-shi,**
**Aichi 4870032 (JP)**

(74) Representative: **Paget, Hugh Charles Edward et al**
**Mewburn Ellis LLP**
**York House**
**23 Kingsway**
**London WC2B 6HP (GB)**

(54) **COMPOSITION FOR OPHTHALMIC USE**

(57) It is a problem of the present invention to provide an ophthalmic composition, wherein: the ophthalmic composition assures a sufficiently high degree of safety to eyes of the lens wearer; the ophthalmic composition reduces symptoms such as an unpleasant feeling or dryness of the eyes; the ophthalmic composition does not adversely affect a contact lens, such as a change of the size of the contact lens; and the ophthalmic composition advantageously improves a water wettability of surfaces of the contact lens, and the ophthalmic composition for the contact lens is prepared by adding at least one specific amino-sugar derivative to an aqueous medium.

EP 1 611 883 A1

## Description

TECHNICAL FIELD

The present invention relates in general to an ophthalmic composition, and more particularly to an ophthalmic composition, which reduces an unpleasant feeling or dryness, while it sufficiently assures a safety to the eye. In particular, the present invention is concerned in eye drops and a solution for a contact lens (hereinafter referred to as "contact lens solution").

BACKGROUND ART

In recent years, there are more and more patients, who complain of dryness or an unpleasant feeling of the eyes, because of wearing of contact lenses or a work with a VDT (Visual Display Terminal).
Conventionally, administration of an artificial tear solution is recommended for the patients, who complain of the above-mentioned symptoms. Dryness can be eased just after the administration of the eye drops, such as the artificial tear solution, but there is a problem that the effect is poor in persistence.
In order to maintain the effect of relief of dryness, there have been devised methods for improving the wettability of the contact lens and for extending a period of time of the eye drops to stay on a cornea, by mixing a thickener etc., to the eye drops, but a sufficient effect cannot be obtained. Moreover, by mixing a lot of thickener to the eye drops, the viscosity of the eye drops becomes excessively high, which results in problems, such as causing an unpleasant feeling at a time of the administration of the eye drops.
Meanwhile, quite a few contact lens wearers are patients, who complain of symptoms such as a cloudiness of the lenses. In addition to this, eye irritation, discomfort due to unclear image etc., and symptoms, such as dryness, are caused, at a time of wearing the contact lenses. For this reason, a cellulose-based or a vinyl-based hydrophilic polymer, or a surfactant, etc., is mixed with the contact lens solution, in order to lower a surface tension of the contact lens, so as to provide the water wettability to the surfaces of the contact lens, which results in reducing the symptoms such as the cloudiness of the contact lens. However, the effect of reducing the symptoms cannot be realized by simply adding the hydrophilic polymer or surfactant, etc., to the contact lens solution. Moreover, there is a problem that the components of the surfaces of the contact lens are replaced with the lacrimal fluid after wearing the contact lens, which leads to restoring the poor water-wettability of the contact lens, in a short period of time.
Moreover, the concentration of a disinfectant of the above-mentioned contact lens solution tends to be increased, so that the safety of the contact lens solution is questionable.
JP-A-2001-322936 discloses an ophthalmic composition, which includes (A) sodium hydrogen carbonate, and (B) at least one compound selected from the group consisting of a lower polyhydric alcohol having not more than five carbon atoms, sugar alcohols, and terpenoids, in order to prevent and ease dryness of the eye (dry eyes), and symptoms such as dryness of the eye and feeling of wrongness are improved by using the components of (A) and (B) together. JP-A-2001-192333 discloses an ophthalmological composition for the contact lens, including (A) an amino acid and/or its salt, and (B) a refreshing agent, which is intended to improve symptoms caused by wearing the contact lens. According to JP-A-2001-192333, if the components of (A) and (B) are used together, reduction of corneal tissues and occurrence of abnormal formation of the corneal tissues are prevented, and discomfort due to incompatibility of the lens with the eye and unpleasantness caused by wearing the lens are reduced. In addition to this, JP-A-2001-122774 discloses an ophthalmological composition for the contact lens, including a refreshing agent and a polyol. JP-A-2001-122774 explains that the use of the ophthalmological composition suppresses adsorption of the refreshing agent on a soft contact lens, while troubles caused by the soft contact lens, such as astheonopia and dryness, are relieved.
Moreover, JP-A-2002-104971 discloses an ophthalmic composition, which includes a menthol glycoside having monosaccharides such as an amino-sugar, which is bonded to the structure of the menthol glycoside. The disclosed ophthalmic composition enhances a refreshing feeling and persistence without increasing eye irritation. However, the menthol glycoside having monosaccharides, which are bonded to the structure of the menthol glycoside, has a menthol as its basic skeleton, so that the structure is clearly different from specific amino-sugar derivative, which is employed by the present invention.

DISCLOSURE OF THE INVENTION

The present invention was developed in the light of the background art situations described above. A problem to be solved by the invention is to provide an ophthalmic composition, wherein: the ophthalmic composition assures a sufficiently high degree of safety to the eyes of the lens wearer; the ophthalmic composition reduces symptoms such as an unpleasant feeling or dryness of the eyes; the ophthalmic composition does not adversely affect the contact lens, such as a change of the size of the contact lens; and the ophthalmic composition advantageously improves a water

wettability of surfaces of the contact lens.

The inventors of the present invention have made an extensive research on the ophthalmic composition in an effort to solve the above-indicated problem, and found that a specific amino-sugar derivative added to the ophthalmic composition advantageously reduces the unpleasant feeling or dryness of the eyes and provides the contact lens with an excellent water wettability, without adversely affecting the contact lens, such as the change of the size of the contact lens. The inventors of the present invention have also found that if the specific amino-sugar derivative is added to the contact lens composition, which includes the disinfectant, the disinfecting effect of the disinfectant is effectively improved, so that the ophthalmic composition can assure high safety to the eyes, without increasing the concentration of the disinfectant.

The above-mentioned problem of the present invention may be solved according to one aspect of the present invention, which provides an ophthalmic composition, characterized by containing, in an aqueous medium, at least one compound of amino-sugar derivative represented by the following general formula (I):

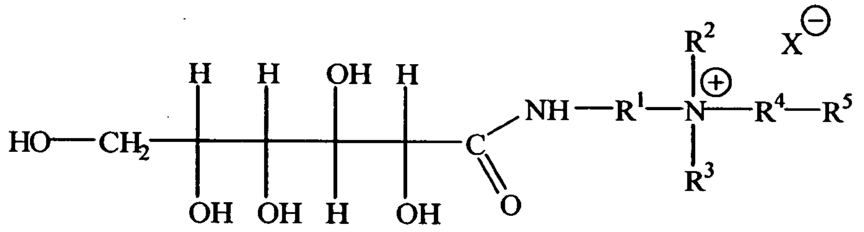

... (I)

wherein, each of $R^1$ and $R^4$ independently represents a divalent hydrocarbon group having 1 - 8 carbon atoms; each of $R^2$ and $R^3$ independently represents a monovalent hydrocarbon group having 1 - 3 carbon atoms; and $R^5$ represents a monovalent hydrocarbon group or OH; and $X^-$ is $OH^-$, $Cl^-$ or $Br^-$.

The present ophthalmic composition described above includes the specific amino-sugar derivative represented by the above general formula (I), as one of components of the ophthalmic composition. Owing to the addition of the specific amino-sugar derivative, the present ophthalmic composition advantageously relieves symptoms such as the unpleasant feeling or dryness caused by the work with VDT, in which the eyes are overstrained, or caused by wearing the contact lens.

In addition to the above, the specific amino-sugar derivative used for the present invention doesn't cause adverse effect such as swelling or shrinking of the contact lens, so that the change of the configuration or the size of the contact lens is advantageously prevented, whereby an excellent compatibility of the ophthalmic composition with the contact lens is realized. Moreover, the specific amino-sugar derivative is also a component, which adheres to or adsorbs on the surfaces of the contact lens, for thereby improving the hydrophilicity of the contact lens and providing excellent water wettability to the contact lens. Therefore, the occurrence of cloudiness etc. of the contact lens is also advantageously prevented.

In addition to the above, if the specific amino-sugar derivative is mixed to the ophthalmic composition such as the contact lens solution, which includes the disinfectant, the disinfecting action of the disinfectant is effectively improved. Accordingly, the present ophthalmic composition advantageously assures a high degree of disinfecting effect even if the concentration of the disinfectant included therein is minimized. Therefore, the present ophthalmic composition can also enjoy the advantage of further enhanced safety to the lens wearer's eyes.

As the examples of the amino-sugar derivative represented by the above general formula (I), there is preferably employed a (gamma-gluconamidopropyl)dimethyl hydroxyethylammonium chloride, wherein $R^1$ is $-CH_2-CH_2-$, each of $R^2$ and $R^3$ is $-CH_3$, $R^4$ is $-CH_2-CH_2-$, $R^5$ is -OH, and $X^-$ is $Cl^-$.

According to a preferred form of the present invention, it is desirable that the amino-sugar derivative in the ophthalmic composition has a concentration within a range of 0.0001 to 10w/w%. By the employment of the amino-sugar derivative within the range of the above-mentioned concentration, the ophthalmic composition can enjoy an excellent effect to ease the symptoms such as the unpleasant feeling or dryness of the eyes.

According to another preferred form of the present invention, it is desirable that the ophthalmic composition further contains the disinfectant. If the disinfectant is added to the ophthalmic composition, the disinfecting effect of the disinfectant can be effectively improved, owing to the above-mentioned amino-sugar derivative. As the disinfectant, a biguanide compound or a polymeric quaternary ammonium compound can be advantageously employed.

According to another preferred form of the present invention, it is desirable that the ophthalmic composition further contains at least one of a preservative, a chelating agent, an isotonic agent, a buffer, a thickener, a surfactant, an antiphlogistic agent, a decongestant, an antiallergenic agent, a refreshing agent, a flavoring substance, vitamins, and amino acids, so that the ophthalmic composition has a further function given by the additional component.

According to another preferred form of the present invention, the present ophthalmic composition can be used as eye drops, because the present ophthalmic composition has a sufficient safety to the eyes. Administration of a prescribed amount of the eye drops can ease the symptoms such as dryness of the eye and the unpleasant feeling. The above-mentioned eye drops do not adversely affect the contact lens, so that it is possible to administrate the eye drops while the contact lens is still worn on the eyes. Accordingly, the hydrophilicity of the surfaces of the contact lens is improved, owing to the above-mentioned amino-sugar derivative, whereby excellent water wettability is given to the contact lens, and the occurrence of the cloudiness of the contact lens can be effectively prevented.

According to another preferred form of the present invention, it is also desirable that the present ophthalmic composition is used as a contact lens solution. If the present ophthalmic composition is used as the contact lens solution, the hydrophilicity of the surfaces of the contact lens is improved, owing to the above-mentioned amino-sugar derivative, whereby excellent water wettability is given to the contact lens, and the occurrence of the cloudiness of the contact lens can be effectively prevented. In addition to this, various symptoms caused by wearing the contact lens, including discomfort, such as eye irritation and difficulty in viewing, and symptoms, such as dryness, are advantageously eased.

## BEST MODE FOR CARRYING OUT THE INVENTION

The present ophthalmic composition is characterized by comprising at least one compound of, or not less than two compounds of specific amino-sugar derivatives. The present ophthalmic composition is also characterized by containing the amino-sugar derivative in an aqueous medium, so as to be able to exhibit an intended advantageous effect.

The specific amino-sugar derivative used in the present invention is an amino-sugar derivative (quaternary ammonium compound) represented by the above general formula (I). By the addition of at least one compound of the specific amino-sugar derivative (quaternary ammonium compound), symptoms, such as the unpleasant feeling and dryness caused by the work with VDT, in which the eyes are overstrained, or by wearing the contact lens, are advantageously reduced, and the effect can be maintained longer than that of the conventionally used ophthalmic composition.

The amino-sugar derivative (quaternary ammonium compound) represented by the above general formula (I) doesn't change the configuration or the size of the contact lens, so that the ophthalmic composition, which contains the amino-sugar derivative (quaternary ammonium compound), has an excellent compatibility with the contact lens.

Moreover, if the contact lens is held in contact with the ophthalmic composition, which contains the amino-sugar derivative (quaternary ammonium compound) represented by the above general formula (I), the amino-sugar derivative is electrostatically adsorbed on or attached to the surfaces of the contact lens, so as to render the surfaces of the contact lens hydrophilic, whereby the excellent water wettability is given to the contact lens.

In the general formula (I), each of $R^1$ and $R^4$ independently represents a divalent hydrocarbon group having 1 ~8 carbon atoms. As the divalent hydrocarbon group having 1~ 8 carbon atoms, there may be used an alkylene group and a phenylene group, and among these groups, an ethylene group and a trimethylene groups having 2 or 3 carbon atoms are desirable.

Each of $R^2$ and $R^3$ independently represents a monovalent hydrocarbon group having 1~3 carbon atoms. As the monovalent hydrocarbon group having 1~3 carbon atoms, there may be used alkyl groups, such as a methyl group, an ethyl group, and a propyl group. Among them, a methyl group having one carbon atom is desirable.

In the above general formula (I), $R^5$ represents a monovalent hydrocarbon group having 1~8 carbon atoms or OH. As the monovalent hydrocarbon group of having 1~8 carbon atoms, an alkyl group and a phenyl group may be used, and among them, OH group is desirable as $R^5$.

In addition to the above, $X^-$ is a monovalent anion, and represents $OH^-$, $Cl^-$ or $Br^-$.

Among the amino-sugar derivative (quaternary ammonium compound) represented by the above general formula (I), there is preferably employed a (gamma-gluconamidopropyl)dimethyl hydroxyethylammonium chloride, which is also known as quaternium-22; wherein $R^1$ is a trimethylene group; each of $R^2$ and $R^3$ is a methyl group; $R^4$ is an ethylene group; $R^5$ is a hydroxyl group; and $X^-$ is a chloride ion. This is because the (gamma-gluconamidopropyl)dimethyl hydroxyethylammonium chloride assures safety to the eyes, and further effectively provide the hidrophilicity to the surfaces of the contact lens. Accordingly, symptoms, such as the unpleasant feeling or dryness, are more effectively eased, and the effect can be maintained for a long period of time. The amino-sugar derivative (quaternary ammonium compound) is commercially obtainable. For instance, (gamma-gluconamidopropyl)dimethyl hydroxyethylammonium chloride is available under a commercial name "Ceraphyl-60" (available from ISP Van Dyk Inc.).

The concentration of the amino-sugar derivative (quaternary ammonium compound) represented by the above general formula (I) in the ophthalmic composition may be suitably determined, in order to advantageously obtain the desired effect of reducing the unpleasant feeling and dryness, and improving the water wettability of the surfaces of the contact lens. If the concentration of the amino-sugar derivative in the ophthalmic composition is excessively low, the effect of the amino-sugar derivative may not be sufficiently obtained. On the other hand, if the concentration is excessively high, it may cause eye irritation or trouble to the eye. In this case, it may also adversely affect the contact lens or the eye when the contact lens is held in contact with the ophthalmic composition, because an excessive amount of the

amino-sugar derivative may be adhered to the surfaces of the contact lens. In view of this, the effective amount of the amino-sugar derivative is generally in a range of 0.0001 to 10 % by weight (w/w%) of the total amount of the ophthalmic composition, preferably, 0.01 to 5 % by weight of the total amount of the ophthalmic composition.

The ophthalmic composition according to the present invention is obtained by adding suitable amount of the amino-sugar derivative to and dissolving the amino-sugar derivative in the aqueous medium, similar to a preparation of conventionally known aqueous medium. The ophthalmic composition may further contain, as needed, various known additives as used in the conventional eye drops or the contact lens solution, in addition to the above-described specific amino-sugar derivative. The additives to be included in the disinfecting liquid should be safe to the living body and ophthalmically acceptable, and should not give adverse influences on the configuration or the physical properties of the contact lens. The additives are preferably included in the ophthalmic composition within an amount that fulfils the above-mentioned requirements. Therefore, various functions provided by the components of the additives can be advantageously added to the ophthalmic composition, without deteriorating the effect of the present invention.

For instance, the ophthalmic composition of the present invention can include a disinfectant, which has a disinfecting or a sterilizing effect, in order to advantageously exhibit the disinfecting effect for the eye and the contact lens, as well as a preservative or conservative effects for the ophthalmic composition.

In particular, the ophthalmic composition of the present invention includes the above-mentioned specific amino-sugar derivative, and the specific amino-sugar derivative effectively improves the disinfecting effect of the disinfectant, so that even if the concentration of the disinfectant in the ophthalmic composition is very low, i.e., within a range of about 0.1 ppm to about 10 ppm, preferably about 0.1 ppm to about 6 ppm, sufficiently high disinfecting effect can be advantageously obtained. In this way, the concentration of the disinfectant in the ophthalmic composition can be limited to be very low, so that the ophthalmic composition assures sufficient safety to the eyes.

It is generally desirable that the disinfectant has the disinfecting effect, and has the excellent compatibility with the contact lens, and which is less likely to cause troubles such as an allergy. Any one of, or any combination of the conventionally known disinfectants may be selected and used. Among these disinfectants, biguanide compound and a polymeric quaternary ammonium compound are especially preferably used in the present invention, because these compounds are known as having especially useful disinfecting characteristic, compared with the other disinfectants, and the disinfecting characteristic of the biguanide compound and the polymeric quaternary ammonium compound can be more effectively improved by the amino-sugar derivative, which is the essential component of the present invention.

Examples of the biguanide compound include polyhexamethylene biguanide (PHMB) and biguanide polymer, which is represented by the following general formula (II):

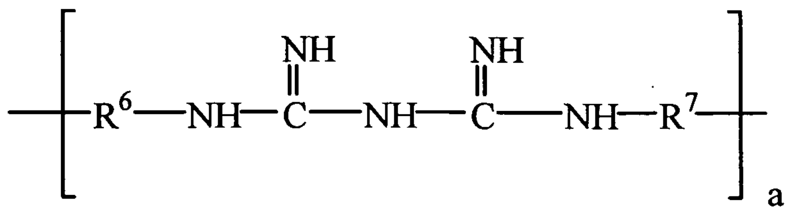 · · · (II)

wherein, a represents an integer of not smaller than 1; and each of $R^6$ and $R^7$ independently represents a divalent group represented by CnHmOp, wherein n=1~24, m=2~48, and p=0~ 11.

Examples of the polymeric quaternary ammonium compound include polydronium chloride (polyquarterium-1), Glokill PQ (commercial name, available from Rhodia Inc.), Unisense CP (commercial name, poly(diallyl dimethyl ammonium chloride, available from SENKA Corporation), WSCP (commercial name, poly[oxyethylene(dimethyliminio)ethylene-(dimethyliminio)ethyl ene dichloride] is included in a concentration of about 60% by weight, available from Buckman Laboratories International, Inc.. In addition to the above, there may also be used polymeric quaternary ammonium compounds, represented by the following general formulas (III) to (V), a condensation product of diamines and a dihalogen compound, as disclosed in the Japanese Patent No. 2550036, and a polycationic compounds as disclosed in JP-A-4(1992)-231054, JP-A-8(1996)-512145, and JP-A-11(1999)-249087, etc..

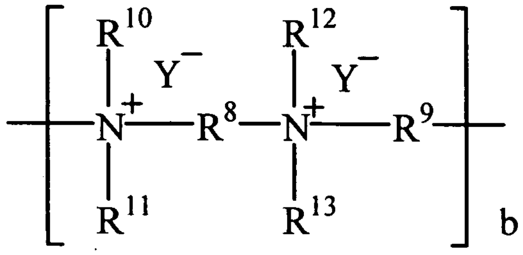

· · · (III)

wherein, b represents an integer of not smaller than 1; $Y^-$ is a monovalent anion such as $Cl^-$; each of $R^8$ and $R^9$ independently represents a divalent group represented by CnHmOp, wherein n=1~24, m=2~48, and p=0~11; and each of $R^{10}$, $R^{11}$, $R^{12}$ and $R^{13}$ independently represents a monovalent group represented by CqHrOs, wherein q=1~4, r=2~9, and s=0~1.

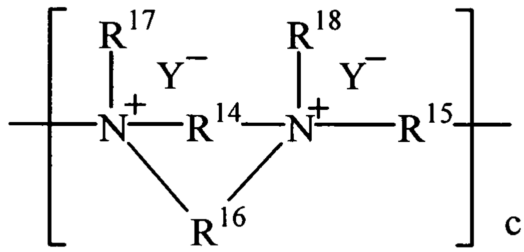

· · · (IV)

wherein, c represents an integer of not smaller than 1; $Y^-$ is a monovalent anion such as $Cl^-$; each of $R^{14}$ and $R^{15}$ independently represents a divalent group represented by CnHmOp, wherein n=1~24, m=2~48, and p=0~11; $R^{16}$ represents a monovalent group represented by CtHuOv, wherein t=1~4, u=2~9, and v=0 ~ 1; and each of $R^{17}$ and $R^{18}$ independently represents a monovalent group represented by CqHrOs, wherein q=1~4, r=2 ~9, and s=0~1.

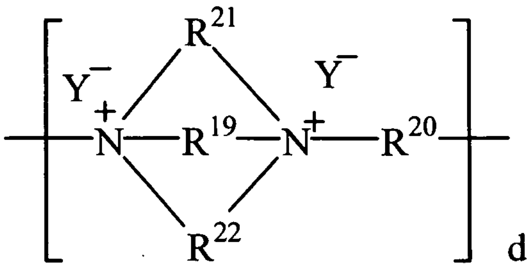

· · · (V)

wherein, d represents an integer of not smaller than 1; $Y^-$ is a monovalent anion such as $Cl^-$; each of $R^{19}$ and $R^{20}$ independently represents a divalent group represented by CnHmOp, wherein n=1~24, m=2~48, and p=0~11; and each of $R^{21}$ and $R^{22}$ independently represents a divalent group represented by CtHuOv, wherein t=1~4, u=2~9, and v=0~1.

In addition to the above-mentioned disinfectant, the ophthalmic composition according to the present invention may further include, as needed, various known additives as used in the conventional eye drops or contact lens solution, such as a preservative, chelating agent, an isotonic agent, a buffer, a thickener, a surfactant, an antiphlogistic agent, a decongestant, an antiallergenic agent, a refreshing agent, a flavoring substance, vitamins, and amino acids, and any one of, or any combination of these additives may be suitably selected and included.

In detail, a preservative may be additionally included in the ophthalmic composition, similar to the above-mentioned disinfectant, so as to exhibit the preservative and conservative effect. Examples of the preservative include sorbic acid, potassium sorbate, benzoic acid or salts thereof, ethyl parahydroxybenzoate, butyl p-hydroxybenzoate, propyl parahydroxybenzoate, methyl parahydroxybenzoate, chlorobutanol, and perbotates such as perboric acid or sodium

perborate.

It is possible that deposits from the lacrimal fluid such as calcium may adhere to the contact lenses, especially, soft contact lenses. Therefore, a chelating agent is also advantageously included in the present ophthalmic composition. Examples of the chelating agent include ethylenediamine tetraacetic acid (EDTA) and salts thereof, such as disodium salts of ethylenediamine tetraacetic acid (EDTA · 2Na) and trisodium salts of ethylenediamine tetraacetic acid (EDTA · 3Na).

Further, if the pH value or the osmotic pressure of the ophthalmic composition according to the present invention is excessively high or low, it may cause eye irritation or other problem to the eye. Therefore, in the ophthalmic composition, the pH value is adjusted to be within a range of 5.3 to 8.5, preferably around 7.0, while the osmotic pressure is adjusted to be within a range of 200 to 400 mOsm/kg, by an addition of the isotonic agent.

Examples of the pH adjusting agent, which is used for adjusting the pH, include sodium hydroxide and hydrochloric acid. The buffer to effectively keep the pH value of the ophthalmic composition within the above-mentioned range, assuring the safety to the eye, is suitably selected among conventionally known various buffers. In particular, examples of the buffer include: acids such as phosphoric acid, boric acid, carboxylic acid, oxycarboxylic acid, and salts thereof (such as sodium salts); a Good-Buffer, tris(hydroxymethyl)aminomethane (TRIS), bis(2-hydroxyethyl)iminotris(hydroxymethyl)methane (Bis-Tris) and sodium hydrogen carbonate. These buffers are selected, because they are safe to the eyes and are able to reduce influences to the contact lenses.

As the isotonic agent, which is used for adjusting the osmotic pressure, at least one compound of sodium chloride, potassium chloride, saccharides, a sugar alcohol, a polyol, and ethers or esters thereof may be generally used.

The present ophthalmic composition may further contain a thickener, in order to suitably adjust the viscosity of the composition, and to extend the period of time for the amino-sugar derivative to stay on the corneal surface. By the addition of the thickener, the components included in the ophthalmic composition can be effectively absorbed, and the effect of reducing dryness etc. of the eye can be exhibited for an extended period of time. Examples of the thickener include: mucopolysaccharides, various gums such as heteropolysaccharides; synthetic organic high molecular compounds such as polyvinyl alcohol, poly-N-vinylpyrrolidone, polyethylene glycol, polypropylene glycol, polyacrylamide; cellulose derivatives such as hydroxy ethyl cellulose, hydroxypropyl methylcellulose, carboxymethyl cellulose, methyl cellulose; and starch derivatives, and these thickeners are advantageously used.

The present ophthalmic composition may further include a known surfactant, in order to advantageously exhibit a removal effect (cleaning effect) with respect to deposits such as eye lipid from the surfaces of the eye and to stably dissolve (solubilize) a non-aqueous component in the aqueous medium. As the surfactant, any known anionic surfactants, nonionic surfactants, amphoteric surfactants, and cationic surfactants may be used in the present invention, as long as they assure a high degree of safety to the living body without adversely affecting the material of the contact lens. The surfactant is added to the present ophthalmic composition in suitable concentrations so as not to adversely influence the effect of the present invention.

Examples of the surfactant include: polyoxyethylne-polyoxypropylene block copolymer and derivatives thereof; polyethylene glycol derivatives of condensation products of polyoxyethylene alkylphenyl ether and formaldehyde, such as tiloxapol; sorbitol fatty acid ester such as sorbitan sesquioleate; polyoxyethylene sorbitan fatty acid ester such as polyoxyethylene sorbitan monooleate (e.g., Polysorbate 80); glycerin fatty acid ester such as glyceryl monostearate; polyethylene glycol fatty acid ester such as polyethylene glycol monostearate; polyoxyethylene alkylether such as polyoxyethylene lauryl ether; and polyoxyethylene hardened castor oil. Among the surfactants, it is especially desirable to use commercially available nonionic surfactants, such as Pluronic, which is a polyoxyethylene-polyoxypropylene block copolymer, Pluronic, Pluronic R, Tetronic, Tetronic R (all available from BASF A.G., Germany). In particular, it is desirable to use Poloxamer 124, Poloxamer 188, Poloxamer 237, Poloxamer 338, Poloxamer 407, Tetronic 904, Tetronic 908, Tetronic 1304, Tetronic 1107, or Polysorbate 80, which is a polyoxyethylene sorbitan monooleate.

In addition to the above, the ophthalmic composition in accordance with the present invention may, if appropriate, include: antiphlogistic agents such as dipotassium glycyrrhizinate, allantoin, berberine chloride, berberine sulfate, sodium azulene sulfonate, zinc sulfate, zinc lactate, and lysozyme; decongestants such as epinephrine, epinephrine hydrochloride, ephedrine hydrochloride, naphazoline hydrochloride, naphazoline nitrate, phenylephrine hydrochloride, and tetrahydrozoline hydrochloride; antihistamines such as diphenhydramine hydrochloride and chlorpheniramine malate; sulfa drugs such as sulfamethoxazole, sodium sulfamethoxazole, sulfisoxazole, and sodium sulfisomidine; antiallergenic agent such as cromolyn, sodium cromoglycate, tranilast, and pemirolast potassium; refreshing agents such as fennel oil, d-camphor, d1-camphor, coolmint No. 71212, geraniol, peppermint water, peppermint oil, bergamot oil, d-borneol, d1-borneol, 1-menthol, d1-menthol, eucalyptus oil, linalool, N-ethyl-p-menthane-3-carboxyamide (for instance, WS-3 available from Takasago International Corporation); flavoring substances such as ascorbic acid, L-aspartic acid, L-sodium aspartate, L-magnesium aspartate, aspartame, hydrangea leaves, extract of hydrangea, powdered hydrangea, aminoethylsulfonic acid, fennel, fennel tincture, powdered fennel, fennel oil, erythritol, licorice, extract of licorice, D-sorbitol, D-sorbitol solution, and glucose; vitamins such as flavin adenine dinucleotide sodium (activated vitamin $B_2$), pyridoxine hydrochloride (vitamin $B_6$), cyanocobalamin (vitamin $B_{12}$), vitamin E acetate, panthenol, calcium pantothenate, sodium pantothenate, retinol acetate, and retinol palmitate (vitamin A palmitate); amino acids such as aspartic acid and salts thereof, aminoethylsulfonic acid, arginine, alanine, lysine, and glutamic acid.

The ophthalmic composition of the present invention is prepared by adding and including respective suitable amounts of the above-mentioned components to the suitable aqueous medium, according to a conventionally known method. It is needles to mention that, in addition to water itself such as a tap water and a purified water, there can also be used any solution, as the aqueous medium, such as a saline solution, a solution which includes sodium chloride, known eye drops, or a contact lens solution, as long as the solution is principally constituted by water.

In preparing the ophthalmic composition of the present invention, which contains the above-mentioned specific amino-sugar derivative, the ophthalmic composition can be easily obtained by simply dissolving the respective components in the aqueous medium, similar to a preparation of conventional solution, without requiring any special method.

The ophthalmic composition of the present invention, which is obtained as described above, assures a sufficient safety to the eye, so that the ophthalmic composition can be advantageously used as the eye drops, or the contact lens solution, for instance. Examples of the contact lens solution include a contact lens disinfecting solution, a contact lens cleaning solution, a contact lens storing solution, and a multipurpose solution (MPS), in which one solution can be used for many purposes such as: cleaning and storing; cleaning, storing, and rinsing; and disinfecting and cleaning.

In administrating the ophthalmic composition of the present invention as the eye drops to the eye, which shows the symptom of dry eye, for instance, suitable amount of the ophthalmic composition can be administrated to the eye, similar to the conventionally known eye drops or eye solution. The specific amino-sugar derivative, which is included in the ophthalmic composition according to the present invention, does not adversely affect the configuration etc. of the contact lens, so that there will be no problem even if the contact lens is still worn on the eye, at a time of administrating the ophthalmic composition. If the eye drops are administrated while the contact lens is still worn on the eye, the hydrophilicity of the surfaces of the contact lens is improved, owing to the eye drops in contact with the contact lens, the water wettability of the surfaces of the contact lens is improved, and the occurrence of the cloudiness etc. of the contact lens can be effectively prevented. In addition to this, the unpleasantness and dryness of the eye caused by the wearing of the contact lens is advantageously reduced, so that the wearing comfort of the contact lens can be significantly improved.

If the ophthalmic composition of the present invention is used as the disinfecting solution for the contact lens, which is one kind of the contact lens solutions, the contact lens, which has been removed from the eye, is placed in a suitable container, which is filled with the ophthalmic composition of the present invention, for thereby disinfecting the contact lens. When it is needed to wear the contact lens again, the contact lens is taken out of the composition, and the contact lens is worn. If the ophthalmic composition of the present invention is held in contact with the contact lens, the above-mentioned specific amino-sugar derivative (quaternary ammonium compound) is adhered to or adsorbed on the surface of the contact lens, whereby the water wettability of the surfaces of the contact lens is improved and the occurrence of the cloudiness etc. of the contact lens can be effectively restrained. In addition to this, the unpleasantness and dryness of the eye caused by the wearing of the contact lens is advantageously reduced, so that the wearing comfort of the contact lens is significantly improved.

If the ophthalmic composition is used as the contact lens solution, the type of the contact lens to be treated is not limited. For example, soft contact lenses, which are classified into non-water-content contact lenses, low-water-content contact lenses, and high-water-content contact lenses, and hard contact lenses can be the contact lenses to be treated with the ophthalmic composition. Therefore, the ophthalmic composition of the present invention is applied to any contact lens, regardless of the material, etc. of the contact lens.

## EXAMPLES

To further clarify the concept of the present invention, some examples of the invention will be described. It is to be understood that the invention is not limited to the details of the illustrated examples and the foregoing description, but may be embodied with various changes, modifications and improvements, which may occur to those skilled in the art without departing from the scope of the invention defined in the attached claims.

Initially, there were prepared various types of ophthalmic compositions (Nos. 1 to 37 of the present inventions and Nos. 1 to 19 of the comparative examples), wherein the pH value of each composition was adjusted to 7.3, by mixing respective amounts of the components, as indicated in the following TABLES 1 to 9. The amount of the sterilized purified water is adjusted, so that the total amount of the above-mentioned components is 100w/w%.

In preparing the ophthalmic composition, there was used (gamma-gluconamidopropyl)dimethyl hydroxyethylammonium chloride (Ceraphyl-60; available from ISP Van Dyk Inc.) as the amino-sugar derivative represented by the above general formula (I), which is the essential component of the present invention. There were used Poloxamer 407 (available from BASF A.G.) as the surfactant, while there was used EDTA · 2Na as the chelating agent. There was used, and as the thickener, hydroxypropyl methylcellulose (Metolose 60SH-4000; available from SHIN-ETSU CHEMICALS, CO., LTD.), while there were used, as the buffer, boric acid and sodium borate, Bis-Tris, tris(hydroxymethyl)aminomethane (Tromethamole, available from Sigma-Aldrich Corporation). As the isotonic agent, propylene glycol and sodium chloride were used. As the pH adjusting agent, hydrochloric acid and sodium hydroxide were used. In addition, there were used, as the disinfectant, polyhexamethylene biguanide (PHMB) and an ionene, which was a quaternary ammonium compound and represented by the following general formula (VI):

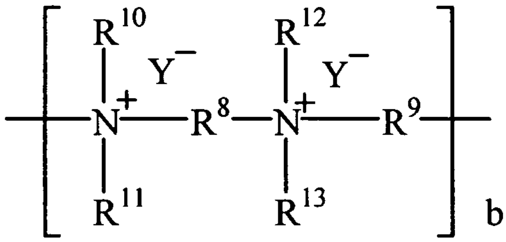

· · · (VI)

wherein b=15, $Y^-=Cl^-$, $R^8=C_6H_{12}$, $R^9=C_6H_{12}$, $R^{10}=CH_3$, $R^{11}=CH_3$, $R^{12}=CH_3$, and $R^{13}=CH_3$.

-Test for examining the disinfecting effect-

9.9mL of each of thus prepared ophthalmic compositions Nos. 1~12 of the present invention and ophthalmic compositions Nos. 1~6 of the comparative examples was put into respective test tubes. To each of the test tubes, there was added 0.1mL of a fungi liquid, which contained C.a. (*Candida albicans* IFO 1594) or S.a. (*Staphylococcus aureus*, IFO 13276) as the test bacteria or fungi in an amount of $10^3$ to $10^9$ cfu/mL, so as to provide a fungi suspension which includes the fungi in an amount of $10^6$ to $10^7$ cfu/mL. The thus obtained specimens were kept at 23 °C for four hours. Thereafter, 1mL of each fungi suspension was taken out of each of the test tubes as a sample, and viable cell count per 1mL of each suspension was measured by using 20mL of Glucose Peptone agar medium, by plate dilution method. On the basis of the obtained value, the viable cell count per 1mL of each fungi suspension was calculated. Then, for each of the ophthalmic compositions, an amount of reduction of the fungi was calculated in logarithm (log reduction) according to the following equation, and the results of the calculation were indicated in the following TABLES 1 to 3:

Log reduction=

log (viable cell count per 1mL of each specimen immediately after inoculation) –

log (viable cell count per 1mL of each fungi suspension after treatment by each contact lens solution specimen)

# TABLE 1

| | | Present Invention | | | | | |
|---|---|---|---|---|---|---|---|
| | | 1 | 2 | 3 | 4 | 5 | 6 |
| Contents [w/w%] | Poloxamer 407 | 0.5 | 0.5 | 0.5 | 0.5 | 0.5 | 0.5 |
| | EDTA · 2Na | 0.05 | 0.05 | 0.05 | 0.05 | 0.05 | 0.05 |
| | Metolose 60SH-4000 | 0.15 | 0.15 | 0.15 | 0.15 | 0.15 | 0.15 |
| | Boric acid | 0.5 | 0.5 | – | – | – | – |
| | Sodium borate | Suitable amount* | Suitable amount* | – | – | – | – |
| | Bis-Tris | – | – | 0.1 | 0.1 | – | – |
| | Tromethamole | – | – | – | – | 0.1 | 0.1 |
| | Propylene glycol | 1.5 | 1.5 | 2.0 | 2.0 | 2.0 | 2.0 |
| | Ceraphyl-60 | 0.5 | 1.0 | 0.5 | 1.0 | 0.5 | 1.0 |
| | PHMB | 0.0001 | 0.0001 | 0.0001 | 0.0001 | 0.0001 | 0.0001 |
| | Ionene | – | – | – | – | – | – |
| | HCl / NaOH | – | – | Suitable amount* | Suitable amount* | Suitable amount* | Suitable amount* |
| Log reduction | *C. albicans* | 2.34 | 2.33 | 2.31 | 2.28 | 2.30 | 2.33 |
| | *S. aureus* | 3.29 | 3.56 | 3.33 | 3.29 | 3.18 | 3.41 |

* Suitable amount to adjust the pH of the mixed composition to 7.3

## TABLE 2

| | | Present Invention | | | | | |
|---|---|---|---|---|---|---|---|
| | | 7 | 8 | 9 | 10 | 11 | 12 |
| Contents [w/w%] | Poloxamer 407 | 0.5 | 0.5 | 0.5 | 0.5 | 0.5 | 0.5 |
| | EDTA · 2Na | 0.05 | 0.05 | 0.05 | 0.05 | 0.05 | 0.05 |
| | Metolose 60SH-4000 | 0.15 | 0.15 | 0.15 | 0.15 | 0.15 | 0.15 |
| | Boric acid | 0.5 | 0.5 | – | – | – | – |
| | Sodium borate | Suitable amount* | Suitable amount* | – | – | – | – |
| | Bis-Tris | – | – | 0.1 | 0.1 | – | – |
| | Tromethamole | – | – | – | – | 0.1 | 0.1 |
| | Propylene glycol | 1.5 | 1.5 | 2.0 | 2.0 | 2.0 | 2.0 |
| | Ceraphyl-60 | 0.5 | 1.0 | 0.5 | 1.0 | 0.5 | 1.0 |
| | PHMB | – | – | – | – | – | – |
| | Ionene | 0.0001 | 0.0001 | 0.0001 | 0.0001 | 0.0001 | 0.0001 |
| | HCl / NaOH | – | – | Suitable amount* | Suitable amount* | Suitable amount* | Suitable amount* |
| Log reduction | *C. albicans* | 2.28 | 2.31 | 2.25 | 2.35 | 2.33 | 2.29 |
| | *S. aureus* | 3.21 | 3.41 | 3.53 | 3.25 | 3.81 | 3.29 |

* Suitable amount to adjust the pH of the mixed composition to 7.3

TABLE 3

| | | Comparative Example 1 | 2 | 3 | 4 | 5 | 6 |
|---|---|---|---|---|---|---|---|
| Contents [w/w%] | Poloxamer 407 | 0.5 | 0.5 | 0.5 | 0.5 | 0.5 | 0.5 |
| | EDTA · 2Na | 0.05 | 0.05 | 0.05 | 0.05 | 0.05 | 0.05 |
| | Metolose 60SH-4000 | 0.15 | 0.15 | 0.15 | 0.15 | 0.15 | 0.15 |
| | Boric acid | 0.5 | – | – | 0.5 | – | – |
| | Sodium borate | Suitable amount* | – | – | Suitable amount* | – | – |
| | Bis-Tris | – | 0.1 | – | – | 0.1 | – |
| | Tromethamole | – | – | 0.1 | – | – | 0.1 |
| | Propylene glycol | 1.5 | 2.0 | 2.0 | 1.5 | 2.0 | 2.0 |
| | Ceraphyl-60 | – | – | – | – | – | – |
| | PHMB | 0.0001 | 0.0001 | 0.0001 | – | – | – |
| | Ionene | – | – | – | 0.0001 | 0.0001 | 0.0001 |
| | HCl / NaOH | – | Suitable amount* | Suitable amount* | – | Suitable amount* | Suitable amount* |
| Log reduction | *C. albicans* | 1.47 | 1.43 | 1.39 | 1.19 | 0.98 | 1.02 |
| | *S. aureus* | 3.87 | 3.92 | 3.76 | 2.89 | 3.01 | 3.18 |

* Suitable amount to adjust the pH of the mixed composition to 7.3

As is apparent from the results in the above TABLES 1 to 3, the ophthalmic compositions Nos. 1~12 of the present invention, which contain the amino-sugar derivative (quaternary ammonium compound) represented by the above general formula (I) exhibited significantly improved disinfecting effect with respect to the *Candida albicans*, compared with the ophthalmic compositions Nos. 1~6 of the comparative examples. The ophthalmic compositions Nos. 1~6 of the present invention, to which PHMB was added as the disinfectant, exhibited a disinfecting effect with respect to the *Staphylococcus aureus*, and the disinfecting effect was almost the same as that of the ophthalmic compositions Nos. 1~3 of the comparative examples, to which the amino-sugar derivative was not added. Moreover, the ophthalmic compositions Nos. 7~12 of the present invention, to which ionene was added as the disinfectant, exhibited effectively improved disinfecting effect with respect to the *Staphylococcus aureus*, compared with the ophthalmic compositions Nos. 4~6 of the comparative examples, to which the amino-sugar derivative was not added.

Therefore, if the amino-sugar derivative (quaternary ammonium compound) represented by the above general formula (I) is added to the ophthalmic composition, the disinfecting effect of the disinfectant is advantageously enhanced, although the effect is slightly varied depending on the kind of the bacteria to be disinfected and the kind of the disinfectant to be used in combination with the ophthalmic composition.

-Test for examining the compatibility with the lens-

Plural numbers of commercially available soft contact lenses (MENICON SOFT MA and MENICON SOFT 72, both available from Menicon Co., Ltd.) and hard contact lenses with high oxygen permeability (MENICON Z and MENICON EX both available from Menicon Co., Ltd.) were obtained, and immerged in a physiological salt solution in accordance with the regulation of ISO 10344, which was kept at 25°C. The diameter of each of the soft contact lenses was measured in a physiological salt solution, by using a projector (Universal Projector, available from Nikon, K.K.). Base curve (radius of curvature) of each of the oxygen permeable hard contact lenses was measured by using a lens meter (CGX-4, an instrument for measurement of contact lens base curve, available from NEITZ INSTRUMENTS, CO., LTD.). Thus obtained values were recorded as initial values of the diameter and the base curve of the lens.

Thereafter, the contact lenses, of which the initial values of the diameters and the base curves had been measured, were immersed in the ophthalmic compositions Nos. 13~21 of the present invention and No. 7~9 of the

comparative inventions at 25°C for one week. Then, the diameter and the base curve of each of the contact lenses were measured by using the same projector or lens meter, as described above. Three contact lenses were subjected to the test for each of the ophthalmic compositions.

Difference (d) between the thus obtained measured value of the diameter or the base curve of each of the contact lens (value after the immersion) and previously obtained initial value of the diameter and the base curve of the contact lens was calculated, according to the equation: d=(value after the immersion) - (initial value). Averages of thus obtained values were also calculated for each of the ophthalmic compositions. It is needless to mention that if the value of the difference of the diameter or the base curve of the contact lens is smaller, the corresponding ophthalmic composition is more excellent in the compatibility with the contact lens. In addition to this, desirable value of the difference of the diameter of the soft contact lens is less than ±0.2 mm, while the desirable value of the difference of the base curve of the oxygen permeable hard contact lens is less than ±0.2 mm. Therefore, the ophthalmic composition, in which the value of the difference of the diameter of the lens was within the above-mentioned range, was evaluated as "successful: ◎", while the ophthalmic composition, in which the value of the difference of the diameter of the lens was out of the above-mentioned range, was evaluated as "unsuccessful: ×", and the results were shown in TABLES 4 and 5 below.

## TABLE 4

| | | Present Invention | | | | | |
|---|---|---|---|---|---|---|---|
| | | 13 | 14 | 15 | 16 | 17 | 18 |
| Contents [w/w%] | Poloxamer 407 | 0.5 | 0.5 | 0.5 | 0.5 | 0.5 | 0.5 |
| | EDTA · 2Na | 0.05 | 0.05 | 0.05 | 0.05 | 0.05 | 0.05 |
| | Metolose 60SH-4000 | 0.15 | 0.15 | 0.15 | 0.15 | 0.15 | 0.15 |
| | Boric acid | 0.5 | 0.5 | 0.5 | 0.5 | 0.5 | 0.5 |
| | Sodium borate | Suitable amount* | Suitable amount* | – | – | – | – |
| | Bis-Tris | – | – | 0.1 | 0.1 | – | – |
| | Tromethamole | – | – | – | – | 0.1 | 0.1 |
| | NaCl | 0.55 | 0.55 | 0.75 | 0.75 | 0.75 | 0.75 |
| | Ceraphyl-60 | 0.5 | 1.0 | 0.5 | 1.0 | 0.5 | 1.0 |
| | PHMB | 0.0001 | 0.0001 | 0.0001 | 0.0001 | 0.0001 | 0.0001 |
| | Ionene | – | – | – | – | – | – |
| | HCl / NaOH | – | – | Suitable amount* | Suitable amount* | Suitable amount* | Suitable amount* |
| Compatibility with contact lens | Soft MA | ◎ | ◎ | ◎ | ◎ | ◎ | ◎ |
| | Soft 72 | ◎ | ◎ | ◎ | ◎ | ◎ | ◎ |
| | MeniconZ | ◎ | ◎ | ◎ | ◎ | ◎ | ◎ |
| | MeniconEX | ◎ | ◎ | ◎ | ◎ | ◎ | ◎ |

* Suitable amount to adjust the pH of the mixed composition to 7.3

# TABLE 5

| | | Present Invention | | | Comparative Example | | |
|---|---|---|---|---|---|---|---|
| | | 19 | 20 | 21 | 7 | 8 | 9 |
| Contents [w/w%] | Poloxamer 407 | 0.5 | 0.5 | 0.5 | 0.5 | 0.5 | 0.5 |
| | EDTA・2Na | 0.05 | 0.05 | 0.05 | 0.05 | 0.05 | 0.05 |
| | Metolose 60SH-4000 | 0.15 | 0.15 | 0.15 | 0.15 | 0.15 | 0.15 |
| | Boric acid | 0.5 | – | – | 0.5 | 0.5 | 0.5 |
| | Sodium borate | Suitable amount* | – | – | Suitable amount* | – | – |
| | Bis-Tris | – | 0.1 | – | – | 0.1 | – |
| | Tromethamole | – | – | 0.1 | – | – | 0.1 |
| | NaCl | 0.55 | 0.75 | 0.75 | 0.55 | 0.75 | 0.75 |
| | Ceraphyl-60 | 1.0 | 1.0 | 1.0 | – | – | – |
| | PHMB | – | – | – | 0.0001 | 0.0001 | 0.0001 |
| | Ionene | 0.0001 | 0.0001 | 0.0001 | – | – | – |
| | HCl / NaOH | – | Suitable amount* | Suitable amount* | – | Suitable amount* | Suitable amount* |
| Compatibility with contact lens | Soft MA | ◎ | ◎ | ◎ | ◎ | ◎ | ◎ |
| | Soft 72 | ◎ | ◎ | ◎ | ◎ | ◎ | ◎ |
| | MeniconZ | ◎ | ◎ | ◎ | ◎ | ◎ | ◎ |
| | MeniconEX | ◎ | ◎ | ◎ | ◎ | ◎ | ◎ |

* Suitable amount to adjust the pH of the mixed composition to 7.3

As is apparent from the results in the above TABLES 4 and 5, it is recognized that the ophthalmic compositions of the present invention, which contains the amino-sugar derivative (quaternary ammonium compound) represented by the above general formula (I), doesn't adversely affect the configuration of the contact lens (diameter and radius of curvature), same as the ophthalmic composition of the comparative examples, which do not contain the amino-sugar derivative. Therefore, the ophthalmic composition of the present invention can be held in contact with the contact lens, so that the ophthalmic composition can be used as the eye drops, which are applicable whether or not the contact lens is still worn on the eye, or can be used as the contact lens solution.

-Test for evaluating the water wettability-

One hard contact lens with high oxygen permeability (MENICON Z or MENICON EX, both available from Menicon Co., Ltd.) was placed on a palm, and three drops of one of the ophthalmic compositions Nos. 22~37 and comparative examples 10~19 were dropped on the contact lens, and the contact lens was cleaned by finger rubbing for fifteen seconds. Thus cleaned contact lens was rinsed with purified water, and then the contact lens was picked up with tweezers, and further rinsed with purified water, which was accommodated in a beaker. Then the contact lens was soaked in an aqueous solution, which contained 0.9% of sodium chloride, and the contact lens was moved up and down for five times and was gently pulled up out of the aqueous solution in succession with the sixth movement. The contact lens was observed with respect to the condition of water adhering to the surfaces of the lens. Ten contact lenses were subjected to the test for each of the ophthalmic compositions Nos. 22~37 and comparative examples 10~19. Each of the contact lenses was scored, according to the following standards of the evaluation. According to total score of ten contact lenses of each of the ophthalmic compositions, the water wettability of each of the ophthalmic compositions was evaluated as follows: ○=the total score is not less than 70; Δ=the total score is not less than 50 and less than 70; ×=the total score is less than 50. Thus obtained results were shown in the following TABLES 6 to 9.

[Criterion of the evaluation]

Score 10: overall the surfaces of the lens is wet.
Score 7: more than 3/4 of the surfaces of the lens is wet.
Score 5: more than half of the surfaces of the lens is wet.
Score 0: only a part of the surfaces of the lens is wet, or the surfaces of the lens repel water.

# TABLE 6

| | | Present Invention | | | | | | |
|---|---|---|---|---|---|---|---|---|
| | | 22 | 23 | 24 | 25 | 26 | 27 | 28 |
| Contents [w/w%] | Poloxamer 407 | 0.5 | 0.5 | 0.5 | 0.5 | 0.5 | 0.5 | 0.5 |
| | EDTA · 2Na | 0.05 | 0.05 | 0.05 | 0.05 | 0.05 | 0.05 | 0.05 |
| | Metolose 60SH-4000 | 0.15 | 0.15 | 0.15 | 0.15 | 0.15 | 0.15 | – |
| | Boric acid | 0.5 | 0.5 | – | – | – | – | 0.5 |
| | Sodium borate | Suitable amount* | Suitable amount* | – | – | – | – | Suitable amount* |
| | Bis-Tris | – | – | 0.1 | 0.1 | – | – | – |
| | Tromethamole | – | – | – | – | 0.1 | 0.1 | – |
| | Propylene glycol | 1.5 | 1.5 | 2.0 | 2.0 | 2.0 | 2.0 | 1.5 |
| | NaCl | – | – | – | – | – | – | – |
| | Ceraphyl-60 | 0.5 | 1.0 | 0.5 | 1.0 | 0.5 | 1.0 | 1.0 |
| | PHMB | 0.0001 | 0.0001 | 0.0001 | 0.0001 | 0.0001 | 0.0001 | 0.0001 |
| | Ionene | – | – | – | – | – | – | – |
| | HCl / NaOH | – | – | Suitable amount* | Suitable amount* | Suitable amount* | Suitable amount* | – |
| Evaluation of water wettability | | ○ | ○ | ○ | ○ | ○ | ○ | ○ |

* Suitable amount to adjust the pH of the mixed composition to 7.3

# TABLE 7

| | | Present Invention | | | | | |
|---|---|---|---|---|---|---|---|
| | | 29 | 30 | 31 | 32 | 33 | 34 |
| Contents [w/w%] | Poloxamer 407 | – | – | – | 0.5 | 0.5 | 0.5 |
| | EDTA · 2Na | 0.05 | 0.05 | 0.05 | 0.05 | 0.05 | 0.05 |
| | Metolose 60SH-4000 | 0.15 | – | – | 0.15 | 0.15 | 0.15 |
| | Boric acid | 0.5 | 0.5 | 0.5 | 0.5 | 0.5 | – |
| | Sodium borate | Suitable amount* | Suitable amount* | Suitable amount* | Suitable amount* | Suitable amount* | – |
| | Bis-Tris | – | – | – | – | – | 0.1 |
| | Tromethamole | – | – | – | – | – | – |
| | Propylene glycol | 1.5 | 1.5 | – | 1.5 | 1.5 | 2.0 |
| | NaCl | – | – | 0.5 | – | – | – |
| | Ceraphyl-60 | 1.0 | 1.0 | 1.0 | 0.5 | 1.0 | 0.5 |
| | PHMB | 0.0001 | 0.0001 | 0.0001 | – | – | – |
| | Ionene | – | – | – | 0.0001 | 0.0001 | 0.0001 |
| | HCl / NaOH | – | – | – | – | – | Suitable amount* |
| Evaluation of water wettability | | ○ | ○ | ○ | ○ | ○ | ○ |

* Suitable amount to adjust the pH of the mixed composition to 7.3

# TABLE 8

| | | Present Invention | | | Comparative Example | | |
|---|---|---|---|---|---|---|---|
| | | 35 | 36 | 37 | 10 | 11 | 12 |
| Contents [w/w%] | Poloxamer 407 | 0.5 | 0.5 | 0.5 | 0.5 | 0.5 | 0.5 |
| | EDTA・2Na | 0.05 | 0.05 | 0.05 | 0.05 | 0.05 | 0.05 |
| | Metolose 60SH-4000 | 0.15 | 0.15 | 0.15 | 0.15 | 0.15 | 0.15 |
| | Boric acid | – | – | – | 0.5 | – | – |
| | Sodium borate | – | – | – | Suitable amount* | – | – |
| | Bis-Tris | 0.1 | – | – | – | 0.1 | – |
| | Tromethamole | – | 0.1 | 0.1 | – | – | 0.1 |
| | Propylene glycol | 2.0 | 2.0 | 2.0 | 1.5 | 2.0 | 2.0 |
| | NaCl | – | – | – | – | – | – |
| | Ceraphyl-60 | 1.0 | 0.5 | 1.0 | – | – | – |
| | PHMB | – | – | – | 0.0001 | 0.0001 | 0.0001 |
| | Ionene | 0.0001 | 0.0001 | 0.0001 | – | – | – |
| | HCl / NaOH | Suitable amount* | Suitable amount* | Suitable amount* | – | Suitable amount* | Suitable amount* |
| Evaluation of water wettability | | ○ | ○ | ○ | △ | △ | △ |

* Suitable amount to adjust the pH of the mixed composition to 7.3

# TABLE 9

| | | Comparative Example | | | | | | |
|---|---|---|---|---|---|---|---|---|
| | | 13 | 14 | 15 | 16 | 17 | 18 | 19 |
| Contents [w/w%] | Poloxamer 407 | 0.5 | – | – | – | 0.5 | 0.5 | 0.5 |
| | EDTA · 2Na | 0.05 | 0.05 | 0.05 | 0.05 | 0.05 | 0.05 | 0.05 |
| | Metolose 60SH-4000 | – | 0.15 | – | – | 0.15 | 0.15 | 0.15 |
| | Boric acid | 0.5 | 0.5 | 0.5 | 0.5 | 0.5 | – | – |
| | Sodium borate | Suitable amount* | Suitable amount* | Suitable amount* | Suitable amount* | Suitable amount* | – | – |
| | Bis-Tris | – | – | – | – | – | 0.1 | – |
| | Tromethamole | – | – | – | – | – | – | 0.1 |
| | Propylene glycol | 1.5 | 1.5 | 1.5 | – | 1.5 | 2.0 | 2.0 |
| | NaCl | – | – | – | 0.5 | – | – | – |
| | Ceraphyl-60 | – | – | – | – | – | – | – |
| | PHMB | 0.0001 | 0.0001 | 0.0001 | 0.0001 | – | – | – |
| | Ionene | – | – | – | – | 0.0001 | 0.0001 | 0.0001 |
| | HCl / NaOH | – | – | – | – | – | Suitable amount* | Suitable amount* |
| Evaluation of water wettability | | △ | × | × | × | △ | △ | △ |

* Suitable amount to adjust the pH of the mixed composition to 7.3

As is apparent from the results in the above TABLES 6 to 9, it is recognized that none of the ophthalmic compositions Nos. 10~19 of the comparative examples, which do not contain the specific amino-sugar derivative ((gamma-gluconamidopropyl)dimethyl hydroxyethylammonium chloride) is evaluated as "○", while all of the ophthalmic compositions Nos. 22~37 of the present invention are evaluated as "○", so that the ophthalmic compositions of the present invention are excellent in the water wettability. Accordingly, it is understood that the hidrophilicity of the surfaces of the contact lens is effectively improved by the (gamma-gluconamidopropyl)dimethyl hydroxyethylammonium chloride. Moreover, as the excellent water wettability of the contact lens is realized, the occurrence of the cloudiness etc. of the contact lens can be effectively prevented. In addition to this, the unpleasantness and dryness of the eye caused by the wearing of the contact lens can be advantageously reduced.

As is apparent from the foregoing description, at least one compound of specific amino-sugar derivative is included in the present ophthalmic composition, so that symptoms, such as the unpleasant feeling or dryness caused by the work with VDT, in which the eyes are overstrained, or by wearing the contact lens are advantageously reduced.

The specific amino-sugar derivative used for the present invention doesn't cause adverse effect, such as swelling or shrinking of the contact lens, so that the change of the shape or the size of the contact lens is advantageously prevented, whereby an excellent compatibility with the contact lens is realized. Moreover, the specific amino-sugar derivative is also a component, which adheres to or adsorbs on the surfaces of the contact lens, for thereby improving the hydrophilicity of the contact lens and providing excellent water wettability to the contact lens. Therefore, the occurrence of the cloudiness etc. of the contact lens can also be advantageously prevented.

In addition to the above, if the disinfectant, such as the biguanide compound or the polymeric quaternary ammonium compound, is added to the ophthalmic composition, which contains the specific amino-sugar derivative, the ophthalmic composition can enjoy the advantage that the disinfecting ability of the disinfectant can be effectively enhanced. Accordingly, the concentration of the disinfectant in the ophthalmic composition can be limited to be low, so that the safety to the lens user's eyes can be significantly enhanced, compared with the conventional ophthalmic compositions.

If the ophthalmic composition is used as the eye drops, symptoms, such as the unpleasant feeling or dryness caused by the work with VDT, in which the eyes are overstrained, or by wearing the contact lens are advantageously

reduced. In particular, if the ophthalmic composition is administrated to the eye, while the contact lens is still worn on the eye, the water wettability of the surfaces of the contact lens is improved, so that there can be enjoyed the advantage of effectively preventing the occurrence of the cloudiness etc. of the contact lens.

In addition to this, if the ophthalmic composition is used as the contact lens solution, the excellent wettability of the contact lens, which has been held in contact with the ophthalmic lens, is realized, so that the symptoms, such as the discomfort due to incompatibility of the lens with the eye and eye irritation, the unpleasantness caused by unclear image etc., and dryness of the eye caused by the wearing of the contact lens are advantageously reduced, whereby the wearing comfort of the contact lens can be significantly improved.

## Claims

1. An ophthalmic composition, **characterized by** comprising, in an aqueous medium, at least one compound of amino-sugar derivative represented by the following general formula (I):

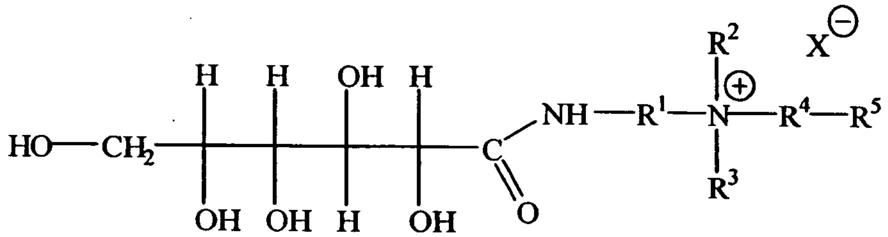

··· (I)

wherein, each of $R^1$ and $R^4$ independently represents a divalent hydrocarbon group having 1~8 carbon atoms; each of $R^2$ and $R^3$ independently represents a monovalent hydrocarbon group having 1 ~ 3 carbon atoms; $R^5$ represents a monovalent hydrocarbon group having 1~8 carbon atoms or OH; and $X^-$ represents $OH^-$, $Cl^-$, or $Br^-$.

2. An ophthalmic composition according to claim 1, wherein said amino-sugar derivative is (gamma-gluconamidopropyl)dimethyl hydroxyethylammonium chloride.

3. An ophthalmic composition according to claim 1 or 2, wherein said amino-sugar derivative in the ophthalmic composition has a concentration within a range of 0.0001 to 10w/w%.

4. An ophthalmic composition according to any one of claims 1 to 3, wherein said ophthalmic composition further comprises a disinfectant.

5. An ophthalmic composition according to claim 4, wherein said disinfectant is one of a biguanide compound and a polymeric quaternary ammonium compound.

6. An ophthalmic composition according to any one of claims 1 to 5, wherein said ophthalmic composition further comprises at least one of a preservative, a chelating agent, an isotonic agent, a buffer, a thickener, a surfactant, an antiphlogistic agent, a decongestant, an antiallergenic agent, a refreshing agent, a flavoring substance, a vitamin preparation, and amino acids.

7. Eye drops, which comprise an ophthalmic composition according to any one of claims 1 to 6.

8. A contact lens solution, which comprises an ophthalmic composition according to any one of claims 1 to 6.

**INTERNATIONAL SEARCH REPORT**

International application No.
PCT/JP2004/001768

A. CLASSIFICATION OF SUBJECT MATTER
Int.Cl[7] A61K31/16, 31/155, 31/785, 9/08, A61P27/04, 31/04, A61L2/18

According to International Patent Classification (IPC) or to both national classification and IPC

B. FIELDS SEARCHED

Minimum documentation searched (classification system followed by classification symbols)
Int.Cl[7] A61K31/16, 31/155, 31/785, 9/08, A61P27/04, 31/04, A61L2/18

Documentation searched other than minimum documentation to the extent that such documents are included in the fields searched

Electronic data base consulted during the international search (name of data base and, where practicable, search terms used)
REGISTRY(STN), CA(STN), BIOSIS(STN), MEDLINE(STN)

C. DOCUMENTS CONSIDERED TO BE RELEVANT

| Category* | Citation of document, with indication, where appropriate, of the relevant passages | Relevant to claim No. |
|---|---|---|
| A | JP 2002-518423 A (The Procter & Gamble Co.), 25 June, 2002 (25.06.02), Claim 4 & WO 99/66888 A1 | 1-8 |
| A | JP 6-107525 A (Helene Curtis, Inc.), 19 April, 1994 (19.04.94), Claim 20 & EP 511652 A1 | 1-8 |
| A | JP 59-88421 A (Richardson-Vicks, Inc.), 22 May, 1984 (22.05.84), Claim 1 & CA 1232204 A | 1-8 |

[X] Further documents are listed in the continuation of Box C.
[ ] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 10 June, 2004 (10.06.04) | 29 June, 2004 (29.06.04) |
| Name and mailing address of the ISA/ Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2004/001768

C (Continuation). DOCUMENTS CONSIDERED TO BE RELEVANT

| Category* | Citation of document, with indication, where appropriate, of the relevant passages | Relevant to claim No. |
|---|---|---|
| P,A | WO 03/066021 A2 (ELAN PHARMA INTERNATIONAL, LTD.), 14 August, 2003 (14.08.03), Claim 14 (Family: none) | 1-8 |

Form PCT/ISA/210 (continuation of second sheet) (January 2004)